# EUROPEAN PATENT SPECIFICATION

(11) **EP 2 254 522 B1**
(45) Date of publication and mention of the grant of the patent: **25.12.2019**
(21) Application number: 09712095.0
(22) Date of filing: 18.02.2009
(51) Int. Cl.: A61B 17/17, A61B 17/80, A61B 17/88

(54) **ORTHOPEDIC PLATES FOR USE IN THE MIDFOOT**
ORTHOPÄDISCHE PLATTEN ZUR VERWENDUNG IM MITTELFUSS
PLAQUES ORTHOPÉDIQUES DESTINÉES À ÊTRE UTILISÉES À MI-PIED

(30) Priority: 19.02.2008 US 66185; 19.02.2008 US 66235; 17.02.2009 US 378540; 17.02.2009 US 378541
(43) Date of publication of application: 01.12.2010
(73) Proprietor: Orthohelix Surgical Designs, Inc., Medina, OH 44256 (US)
(72) Inventor: DUCHARME, Dustin, Stow Ohio 44224 (US); DEN HARTOG, Bryan, D., Rapid City South Dakota 57702 (US); KAY, David, B., Akron Ohio 44333 (US); LEITHER, Andrew, J., San Diego, CA 92122 (US)
(74) Representative: Lavoix
(86) International application number: PCT/US2009/001012
(87) International publication number: WO 2009/105201

(56) References cited:
- WO-A1-98/34553
- US-A1- 2005 059 971
- US-A1- 2006 025 772
- US-A1- 2006 173 459
- US-A1- 2007 016 205
- US-A1- 2007 233 114

## Description

### FIELD OF THE INVENTION

The present invention relates to an orthopedic plate which is configured for the fixation of a bone or bones of the midfoot including, for example, stabilization of a fracture, dislocation or reconstruction of a deformity.

### BACKGROUND OF THE INVENTION

Together the foot and ankle have over 25 bones and 33 joints along with more than 100 named muscles, tendons, and ligaments and a network of blood vessels, nerves, all residing beneath a relatively slim covering of soft tissue and skin. Structurally, the foot has three main anatomical regions: the forefoot, the midfoot, and the hindfoot. These parts work together with the ankle, to provide the body with support, balance, and mobility. A structural flaw or malfunction in any one part can result in the development of problems, which are manifested in other areas of the body.

The forefoot includes the five toes (which are also known as the "phalanges") and their connecting long bones (or "metatarsals"). Several small bones together comprise a phalanx or toe. Four of the five toes have three phalanx bones respectively connected by two joints. The big toe (or "hallux") has two phalanx bones distal and proximal with a joint in between called the interphalangeal joint. The big toe articulates with the head of the first metatarsal at the first metatarsophalangeal joint (the "MTP" joint) and there are two tiny, round bones called sesamoids on the plantar side of the metatarsal head. The phalanges are connected to the metatarsals at the ball of the foot. The forefoot balances pressure on the ball of the foot and bears a substantial amount of the body weight.

The bones of the midfoot from medial to lateral are the 1^{st} through 3^{rd} cuneiform, the cuboid, and the crescent shaped navicular bone posterior to the cuneiforms, which also forms a joint with the talus that forms the basis for the ankle joint at the hinged intersection of the tibia, the fibula, and the foot. The five tarsal bones of the midfoot act together form a lateral arch and a longitudinal arch which help to absorb shock. The plantar fascia (arch ligament) underlays the bones of the midfoot and along with muscles, forms a connection between the forefoot and the hindfoot. The toes and their associated midfoot bones form the first through fifth rays beginning with the great toe as the first ray.

The hindfoot is composed of three joints (subtalar, calcaneocuboid & talonavicular) and links the midfoot to the ankle. The heel bone (or "calcaneus") projects posteriorly to the talus and forms a lever arm to activate the hinged action of the foot so as to allow propulsion of the entire body from this joint. The calcaneus is joined to the talus at the subtalar joint.

The mid-foot is often the subject of trauma such as results from falls, vehicle crashes and dropped objects. These accidents often result in severe fractures and/or dislocations. A common midfoot fracture is the Lisfranc injury which was identified by a French doctor in the Napoleonic Wars. It commonly occurred when a cavalier fell from his horse with his foot caught in his stirrup and resulted in the fracture and dislocation of multiple bones of the midfoot. A Lisfranc injury has come to indicate an injury to the normal alignment of the cuneiforms and metatarsal joints with the loss of their normal spatial relationships. These types of injuries may occur from dropping a heavy object on the top of the foot or stepping on an uneven surface and falling with the foot in a twisted position. These fractures also occur in athletes when the foot is bound to an article of sports equipment such as skis or snowboards or when the foot is subject to simultaneous impact and rotation, such as skating or ballet jumps or soccer.

A common Lisfranc injury occurs at the joint primarily involving the 1st and 2nd metatarsals and the medial cuneiform. Normal alignment of the joints is lost if the ligaments are disrupted and the bones separate between the medial and mid-cuneiforms or between the 1st, 2nd metatarsal and the medial cuneiform. Failure to treat such an injury may result in joint degeneration and subsequent damage to the adjacent nerves and blood vessels.

Typical surgical treatment of the midfoot re-establishes the normal anatomy of the mid-foot while the fractured bones mend. In some cases, fusion of the joint between the first and second metatarsals and the middle and/or internal cuneiforms may be necessary, for example, where arthritis arises in patients with a prior Lisfranc or similar injury. One current surgical treatment of this injury requires that pins, wires and/or screws be inserted to stabilize the bones and joints and hold them in place until healing is complete. For example, a pin or screw may be introduced medially into the internal cuneiform and through the base of the second metatarsal bone. While the use of k-wires, pins, and screws may provide acceptable results for younger and more plastic patients, these methods of fixation are not always satisfactory.

US 2006/0025772 A1 discloses a carpal fusion plate for use in fusion of the bones of the midcarpus or other bones. The fusion plate is concave and defines at least one round countersunk screw hole and at least one tapered elongate slotted aperture. The tapered elongate slotted aperture is narrower peripherally and broader centrally. The fusion plate also defines a multilobated central opening.

### SUMMARY OF THE INVENTION

According to the invention, the plate system according to claim 1 is provided.

In accordance with the present invention an orthopedic plate is provided that is a handy and elegant little plate that can be used in place of a compression staple with better pull-out values, improved compression, and less harm to the bone in which it is used. The invention can be used in a wide variety of indications including for example, lapidus bunionenctomy, calcaneolocuboid fusion, talonavicular fusion, MTP fusion, cuboid fracture, metarsocunieform fusion, chevron osteotomy, Naviculocuneioform fusion, Dwyer osteotomy, cotton osteotomy, isolated TMT fusion, Navicular fracture, Evans osteotomy and the previously mentioned Lisfranc fracture. The present invention is further specifically configured for implantation at the mid-foot In the first embodiment, the plate has a dumbbell or peanut shaped, footprint which consists simply of two rounded tabs along the longitudinal axis of the plate with a narrowed waist section between the tabs. The longer of the two tabs includes a compression slot that extends in a direction and causes compression toward a locking screw hole in the opposing tab. The plate is radiused along the bottom surface along the longitudinal axis of the plate so that the plate forms a section of a cylinder, which is bilaterally symmetrical with respect to a plane passing through the longitudinal axis.

A second embodiment of the plate laterally joins two of the previous plates whereby the locking holes and the compression slots are offset relative to each other. In this embodiment, the plate can have a first radius along the x-axis in the y direction and a second radius along the y-axis in the y direction. The plate has an ring-shaped footprint with two diagonal sets, (one set of longer and one set of shorter) of opposing tabs. In each set of tabs, one tab includes a compression slot that extends and causes compression in a direction toward a screw hole in the opposing tab of the other set of tabs. The maximum length of the plate extends between the longer set of tabs, and an opening is included along this axis to allow viewing of the bone/bone fragments under the plate. The placement of the compression slots and the screw holes account for the progressively anterior placement of the cuneiforms and provides for compression in the medial direction. In a further embodiment, the four tab plate is flat or includes a crease midway between the first and second tabs, which facilitates placement of the plate at the joint between the metatarsals and the cuneiforms.

### BRIEF DESCRIPTION OF THE DRAWINGS

Figure 1 is a dorsal view of a mid-foot with an orthopedic plate in accordance with the invention is positioned for the third ray;
Figure 2 is a top view of the orthopedic plate of Figures 1;
Figure 3 is a side view of the plate shown in Figure 2;
Figure 4 is an end view of the plate shown in Figure 3;
Figure 5 is a top perspective view of the plate shown in Figure 2;
Figure 6 is a end perspective view of the plate shown in Figure 2;
Figure 7 is a bottom perspective view of the plate shown in Figure 2; and
Figure 8 is a side perspective view of the plate shown in Figure 2;
Figure 9 is a dorsal view of a mid-foot with an orthopedic plate in accordance with a second embodiment of the invention is positioned for the first and second ray;
Figure 10 is a dorsal view of a mid-foot with an orthopedic plate in accordance with the second embodiment of the invention is positioned for the second and third ray;
Figure 11 is a perspective view of the orthopedic plate shown in Figures 9 and 10;
Figure 12 is a top view of the orthopedic plate of Figures 9 and 10;
Figure 13 is a first cross-sectional view of the plate shown in Figure 12, taken along line 13-13;
Figure 14 is a second cross-sectional view of the plate shown in Figure 12 taken along line 14-14;
Figure 15 is top view of a further embodiment of an orthopedic plate in accordance with the invention;
Figure 16 is a first side view of the plate shown in Figure 15;
Figure 17 is a second side view of the plate shown in Figure 15;
Figure 18 is a top view of a further embodiment of the invention;
Figure 19 is a side view of the embodiment shown in Figure 20;
Figure 20 is a top view of yet a further embodiment of the invention;
Figure 21 is a perspective view of the embodiment shown in Figure 20; and
Figure 22 is a side perspective view of the embodiment shown in Figure 11 showing a detail of the compression slots.

### DETAILED DESCRIPTION OF THE INVENTION

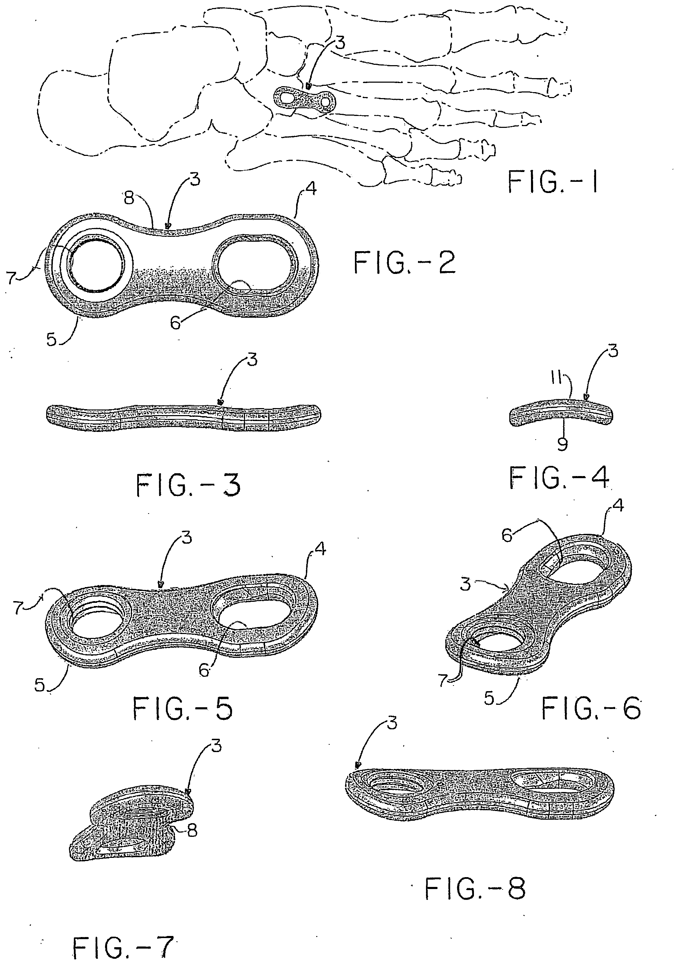

Figure 1 shows a skeletal version of a foot from the top (i.e. the dorsal view) with the midfoot plate 3 of the present invention in place between the junction of the third metatarsal and the third cuneiforms (i.e. the lateral cuneiform). Thus, Figure 1 illustrates the plate used in fixation of the bones of the third ray or 3^{rd} TMT (tarso-metatarsal) joint. The plate can also be used for fixation of the first and second ray, that is, for fixation of the 1^{st} TMT joint (1^{st} metatarsal to medial cuneiform) and 2^{nd} TMT joint (2^{nd} Metatarsal to middle or intermediate cuneiform). Similarly, it can be used for fixation of the joints of the other rays, for example where a surgical staple might currently be used.

As viewed from the top in Figure 2, also in Figures 3 to 8 it can be seen that the plate 3 has two opposing tabs comprising a longer tab 4 and a second shorter tab 5 aligned along the longitudinal axis of the plate. The longer tab 4 includes a compression slot 6, and the other of the pair of tabs includes a screw hole 7 (which preferably includes locking means such as internal threads or a variable locking mechanism, so as to form a locking interface between the plate and the respective bone or bone fragment by means of the rigid fastening of the screw in the screw hole in the plate.) The compression slot is configured so as to cause compression along the longitudinal axis of the plate in the direction of the locking screw hole. The plate includes incurvatures 8 between the tabs to form a waist section which minimizes the material used and maximizes the fit of the plate, as well as allowing additional contouring of the plate in this area, should it be desired.

Figures 3 and 4 illustrate the edge on views of the plate in along a first length and along the second shorter length which is perpendicular to the first length. As can be seen the plate has a generally uniform thickness between the inward surface 9 which opposes and optimally, but not necessarily engages the bones, and the outward surface 11. In addition, the inward surface 9 of the plate 3 includes a generally uniform radius of curvature along the longitudinal axis. Thus, the plate has the shape of a segment of a cylinder which maximizes the ability to place the plate as desired without the need for additional pre-surgical contouring, although the plate thickness allows for bending if necessary

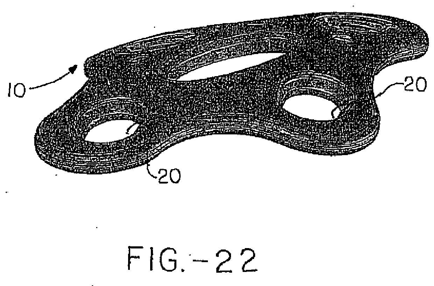

The screws useful with the plate of the present invention are self-starting, self-tapping screws including the option of partial or full cannulation. The screws include a cutting end having multiple flutes, and preferably 2 or 3 flutes about a conical recess. The screws further include a partial taper of the inner diameter in the proximal end over the first several thread turns, for example over 2-8, and preferably over 3-5 turns in order to increase the fatigue life of the screw as well as providing potential physiological advantages in use. The screws further include a torque driving recess. The screws have a threaded distal end and a head including a torque driving recess. The head of the locking screw includes locking means, such as a variable locking mechanism, which could be a bushing that mates with the screw head so as to lock the screw relative to the plate at a desired angle, or could include external screw threads that mate with internal threads in the locking screw hole at a pre-selected angle, in this instance, the screw axis is perpendicular to the longitudinal axis of the plate. The screw used in the compression slot has a rounded rear shoulder (such as a hemisphere, or a torroid) which mates with the concavely rounded groove of the compression slot. The lateral edge of the compression slot further includes an inclined shoulder that slope downward toward the bone-contacting surface of the plate which is engaged by the screw to cause the translation so as to maximize surface contact between the screw head and the inclined geometry of the compression slot. (see Figure 22) The lateral edge of the compression slot further includes an inclined shoulder that slopes downward toward the bone-contacting surface of the plate and which is engaged by the screw head to cause the translation of the screw and attached bone fragment along the long axis of the slot and towards the locking hole.

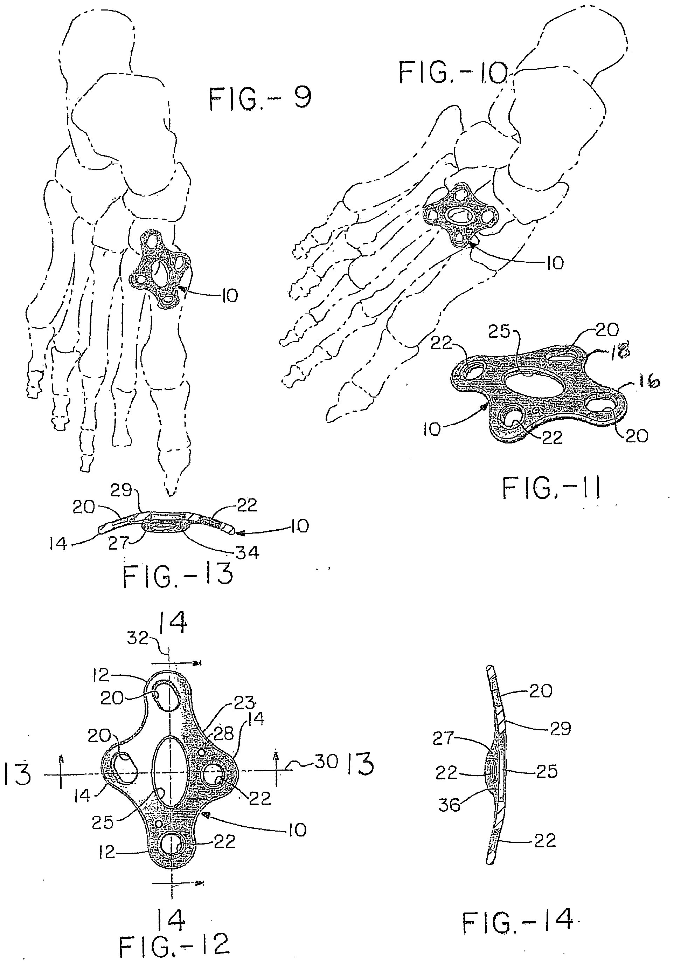

Figure 9 shows a skeletal version of a foot from the top (i.e. the dorsal view) with the four tab embodiment of the midfoot plate 10 of the present invention in place between the junction of the first and second metatarsals and the first and second cuneiforms (i.e. the medial and intermediate cuneiforms). Thus, Figure 9 illustrates the plate used in fixation of the bones of the first and second ray or 1-2 TMT joint complex (1^{st} tarso-metatarsal joint and 2^{nd} tarso-metatarsal joint). The plate can also be used for fixation of the second and third ray (or 2-3 TMT joint complex), i.e. at the base of the second and third metatarsal and the junction of the second and third cuneiforms (i.e. intermediate and lateral cuneiforms) as in shown in Figure 10.

As viewed from the top in Figure 12, it can be seen that the plate 10 has two sets of opposing tabs that are slightly offset from each other so as to define a kind of amemboid shape (i.e. a rhomoid with curving lateral edges that form rounded tabs at the corners). The outline or profile can also be viewed as a modified ring having a first set of diagonally opposed longer tabs 12 and a second set of diagonally opposed shorter tabs 14. Each 16, 18 of one of the longer and the shorter tabs include a compression slot 20, and the other of the pair of tabs includes a screw hole 22 (which can include internal threads so as to form a locking interface with the respective bone or bone fragment.)

The plate 10 includes incurvatures 23 between the tabs to minimize the material used and maximize the fit. At the intersection of the tabs, the plate includes an opening 25 which can be used to view the placement of the plate relative to the bones as well as for adding bone graft material. The opening is preferably an oval shape, which allows the maximization of the area viewed while maintaining sufficient stiffness to hold the bones in position to allow fusion. Holes 28 are provided for k-wires, which can be used to hole the plate in position.

Figures 13 and 14 illustrate the plate in cross-section along a first axis and a perpendicular axis. As can be seen the plate has a generally uniform thickness between the inward surface 27 which opposes and optimally, but not necessarily engages the bones, and the outward surface 29. In addition, while the inward surface 27 of the plate 10 includes a generally uniform radius of curvature 34, 36 along both the first 30 and the second axis 32, the radii of these two curves differ (specifically the radius of the first curvature 34 is from about 50 to about 90, more preferably about 60 to about 80, and most preferably about 65 to about 75 millimeters, while the radius of the second curvature 36 is from about 10 to about 50, more preferably about 20 to about 40, and most preferably about 25 to about 35 millimeters, The first radius is from about 1 to about 4 times that of the second radius, and more preferably about 1.5 to about 3 times that of the second. Thus, the plate has the shape of a segment of a torroid which maximizes the ability to place the plate as desired without the need for additional pre-surgical contouring, although the plate thickness allows for bending if necessary. In a second embodiment, the plate has the same footprint, but is planar or flat.

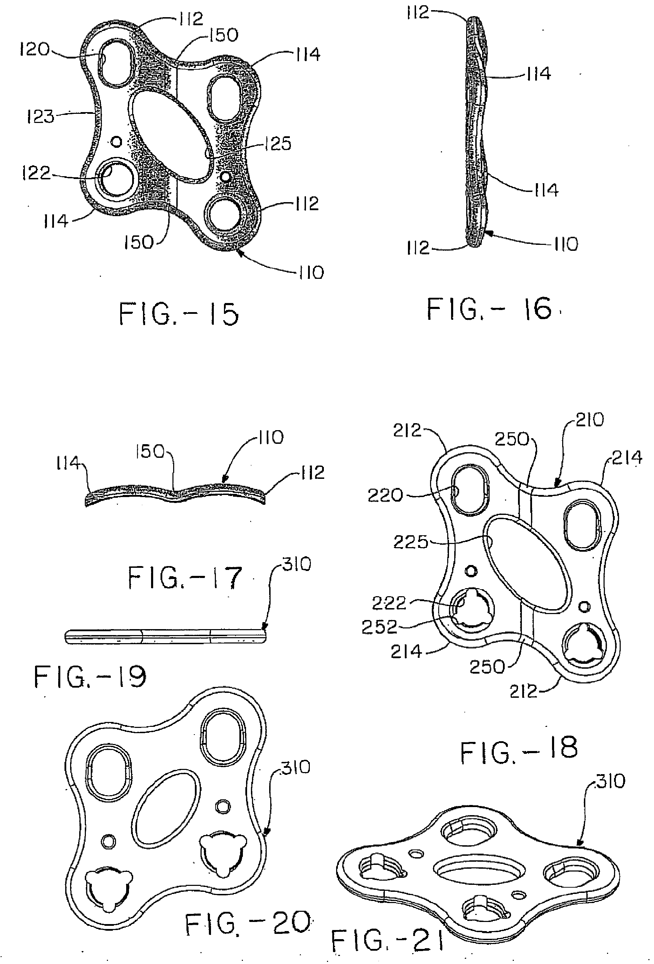

As viewed from the top in Figure 15 also in Figures 16 and 17 it can be seen that the four tab embodiment of the plate 110 also has two sets of opposing tabs that are slightly offset from each other so as to define a kind of amemboid shape (i.e. a rhomoid with curving lateral edges that form rounded tabs at the corners). This embodiment differs from the previous four tab version in that it includes a central "crease" or rib 150 (250) which can be used to help position the plate relative to the bones. A variation of this embodiment is shown in Figure 18, which includes keyway guides 252 in the locking screw holes 220. In the following description, the elements shown in Figure 18 are called out parenthetically following the corresponding elements from Figures 15-17. Again, the outline or profile of the plate 210 can be viewed as a modified ring having a first set of diagonally opposed longer tabs 112 (212) and a second set of diagonally opposed shorter tabs 114 (214). Each one of the longer and the shorter tabs include a compression slot 120 (220), and the other of the pair of tabs includes a screw hole 122 (222) (which can include internal threads so as to form a locking interface with the respective bone or bone fragment.) Alternatively, the screw hole can accommodate other screw/plate interfaces such as a variable locking mechanism to allow the screw to be placed with the axis at a variety of angles relative to the plate and to be subsequently locked into position. One such mechanism is a bushing which is rounded on the outside to swivel in the correspondingly rounded plate hole and which has a locking interface with the head of the locking screw including, for example, mating threads, cams or grooves. The plate includes incurvatures 123 between the tabs to minimize the material used and maximize the fit. At the intersection of the tabs, the plate includes an opening 125 (225) which can be used to view the placement of the plate relative to the bones as well as for adding bone graft material. The opening is preferably an oval shape, which allows the maximization of the area viewed while maintaining sufficient stiffness to hold the bones in position to allow fusion.

In a further embodiment of the four tab plate shown in Figures 19-21, the plate 310 has the same footprint, but is planar or flat in the y direction. These views also illustrate the use of keyways which are cut vertically into the internal threads with in the locking hole. These keyways interface with corresponding vertical hemi-cylinders on a drill guide so as to orient the holes for the locking screws which screw into the locking holes.

The plate is formed of a biocompatible material, and preferably a metal such as surgical grade stainless steel, titanium or a titanium alloy. Preferably, the plate has a thickness of between about 1.0 and 2.0 millimeters, more preferably between about 1.25 and 1.75 millimeters, and most preferably between about 1.4 and 1.6 millimeters. The plate includes a continuous outer edge which is defined between the top and the bottom surface.

In addition, the plate can include a small through hole sized to receive a K-wire or other similar guide wire.

During the surgery the joints are first prepped which may include de-articulation between the bones to be fused. The proper length plate is selected and as necessary, the plate is bent to contour to the bone surface. The plate is placed and held in place using olive wires (thru compression slot and into the bone). The plate is located such that all of the screws are aimed into the targeted bones and away from the joint, fracture, or bone interface. A pilot hole is drilled, for example using a drill guide such as a guide including keyway guides (i.e. lobes) that interlock with corresponding keyway openings in the locking screw hole. The locking screw is tightly screwed into the bone. The olive wire is removed if used, and a pilot hole is drilled at the end of the compression slot farthest away from the fusion or fracture and locking hole. A non-locking screw is inserted into the pilot hole and tightened. As the screw is tightened in the compression slot, it will drive compression toward the fusion site and locking hole. The plate allows for up to 1.5 millimeter of compression. The plate is viewed radiographically, and the soft tissues are closed in the usual manner.

## Claims

1. A plate system for use in associated bone comprising a locking screw and a non-locking screw having a head, and a plate (10, 110, 210) configured for the fixation of a bone or bones of the midfoot, the plate (10, 110, 210) having a top surface (29) and a bottom surface (27) with a generally uniform thickness therebetween, **characterized in that** the plate (10, 110, 210) has a profile which comprises a first lateral side joined to a second lateral side, wherein each of the first lateral side and the second lateral side has a profile which comprises of a first end tab (12, 14, 112, 114, 212, 214) which defines a portion of a circle and a second end tab (12, 14, 112, 114, 212, 214) which defines a portion of a circle wherein the first and second end tabs (5, 12, 14, 112, 114, 212, 214) are aligned along a longitudinal axis of the plate and joined by a middle section, the first end tab (12, 14, 112, 114, 212, 214) including a locking hole (22, 122, 222) for a locking screw, and the second end tab (12, 14, 112, 114, 212, 214) including a compression slot (20, 120, 220) having a long axis, the compression slot (20, 120, 220) including a lateral edge which include an inclined shoulder that slopes downward toward the bottom surface of the plate which is engaged by the non-locking screw head to cause the translation of the non-locking screw along the long axis of the compression slot (20, 120, 220) and towards the locking hole (22, 122, 222), the locking hole (22, 122, 222) and the compression slot (20, 120, 220) being aligned along the longitudinal axis and the non-locking screw interacting with the compression slot to cause compression in the associated bone toward the locking hole in use, and the plate optionally including at least one other hole for a k wire, wherein the plate includes incurvatures (23) between the first end tab and the second end tab to form a waist section, wherein the locking hole (7, 22, 120, 220) includes internal threads and the locking screw has a head having mating external threads.

2. A plate system as set forth in claim 1 wherein the bottom surface (27) is capable of facing a bone and includes a radius of curvature along the longitudinal axis.

3. A plate system as set forth in claim 2 wherein the plate (10) comprises a portion of a cylinder.

4. A plate system as set forth in claim 1 wherein the locking screw includes a variable locking mechanism.

5. A plate system wherein the plate has a profile which is a rhomboid.

6. A plate system as set forth in claim 1 wherein the plate has a longitudinal axis and the bottom surface is capable of facing a bone and includes a radius of curvature along the longitudinal axis.

7. A plate system as set forth in claim 6 wherein the plate has an axis transverse to the longitudinal axis which also includes a radius of curvature.

8. A plate system as set forth in claim 1 wherein the compression slot includes a lateral edge which slopes from the top surface toward the bottom surface in the direction of the locking hole.

9. A plate system as set forth in any one of the preceding claims, wherein the plate has two diagonal sets of opposing tabs (12, 14, 112, 114, 212, 214), wherein the maximum length of the plate extends between the longer set of tabs of the two diagonal sets of opposing tabs.

10. A plate system as set forth in any one of the preceding claims, wherein the locking holes (22, 122, 222) and the compression slots (20, 120, 220) of the first lateral side and the second lateral side are offset relative to each other.

## Patentansprüche

1. Plattensystem zur Verwendung in einem zugeordneten Knochen, eine Verriegelungsschraube und eine nicht-verriegelnde Schraube mit einem Kopf und eine für die Fixierung eines Knochens oder mehrerer Knochen des Mittelfußes ausgelegte Platte (10, 110, 210) umfassend, wobei die Platte (10, 110, 210) eine Oberseite (29) und eine Unterseite (27) mit einer im Allgemeinen gleichmäßigen Dicke dazwischen aufweist, **dadurch gekennzeichnet, dass** die Platte (10, 110, 210) ein Profil aufweist, das eine erste laterale Seite umfasst, die mit einer zweiten lateralen Seite verbunden ist, wobei sowohl die erste laterale Seite als auch die zweite laterale Seite ein Profil aufweisen, das eine Lasche (12, 14, 112, 114, 212, 214) an einem ersten Ende, die einen Abschnitt eines Kreises definiert, und eine Lasche (12, 14, 112, 114, 212, 214) an einem zweiten Ende, die einen Abschnitt eines Kreises definiert, umfasst, wobei die Laschen (5, 12, 14, 112, 114, 212, 214) am ersten Ende und am zweiten Ende entlang einer Längsachse der Platte ausgerichtet sind und durch einen mittleren Abschnitt verbunden sind, wobei die Lasche (12, 14, 112, 114, 212, 214) am ersten Ende ein Verriegelungsloch (22, 122, 222) für eine Verriegelungsschraube aufweist und die Lasche (12, 14, 112, 114, 212, 214) am zweiten Ende einen Kompressionsschlitz (20, 120, 220) mit einer Längsachse aufweist, wobei der Kompressionsschlitz (20, 120, 220) eine laterale Kante aufweist, die einen schrägen Absatz aufweist, der zur Unterseite der Platte hin abfällt und der vom Kopf der nicht-verriegelnden Schraube in Eingriff genommen wird, um die Translation der nicht-verriegelnden Schraube entlang der Längsachse des Kompressionsschlitzes (20, 120, 220) und zum Verriegelungsloch (22, 122, 222) zu bewirken, wobei das Verriegelungsloch (22, 122, 222) und der Kompressionsschlitz (20, 120, 220) entlang der Längsachse ausgerichtet sind und die nicht-verriegelnde Schraube bei Gebrauch mit dem Kompressionsschlitz interagiert, um eine Kompression im zugeordneten Knochen hin zum Verriegelungsloch zu bewirken, und die Platte optional mindestens ein anderes Loch für einen K-Draht aufweist, wobei die Platte Einwärtskrümmungen (23) zwischen der Lasche am ersten Ende und der Lasche am zweiten Ende aufweist, um einen verschmälerten Abschnitt zu bilden, wobei das Verriegelungsloch (7, 22, 120, 220) ein Innengewinde aufweist und die Verriegelungsschraube einen Kopf mit einem passenden Außengewinde aufweist.

2. Plattensystem nach Anspruch 1, wobei die Unterseite (27) in der Lage ist, einem Knochen zugewandt zu werden, und einen Krümmungsradius entlang der Längsachse aufweist.

3. Plattensystem nach Anspruch 2, wobei die Platte (10) einen Abschnitt eines Zylinders umfasst.

4. Plattensystem nach Anspruch 1, wobei die Verriegelungsschraube einen variablen Verriegelungsmechanismus aufweist.

5. Plattensystem, wobei die Platte ein Profil aufweist, das rautenförmig ist.

6. Plattensystem nach Anspruch 1, wobei die Platte eine Längsachse aufweist und die Unterseite in der Lage ist, einem Knochen zugewandt zu werden, und einen Krümmungsradius entlang der Längsachse aufweist.

7. Plattensystem nach Anspruch 6, wobei die Platte eine Achse aufweist, die quer zur Längsachse verläuft, und ebenfalls einen Krümmungsradius aufweist.

8. Plattensystem nach Anspruch 1, wobei der Kompressionsschlitz eine laterale Kante aufweist, die von der Oberseite zur Unterseite in Richtung des Verriegelungslochs schräg abfällt.

9. Plattensystem nach einem der vorangehenden Ansprüche, wobei die Platte zwei diagonale Sätze einander gegenüberliegender Laschen (12, 14, 112, 114, 212, 214) aufweist, wobei sich die maximale Länge der Platte zwischen dem längeren Satz von Laschen von den zwei diagonalen Sätzen einander gegenüberliegender Laschen erstreckt.

10. Plattensystem nach einem der vorangehenden Ansprüche, wobei die Verriegelungslöcher (22, 122, 222) und die Kompressionsschlitze (20, 120, 220) der ersten lateralen Seite und der zweiten lateralen Seite in Bezug aufeinander versetzt sind.

## Revendications

1. Système de plaque destiné à être utilisé dans un os associé comprenant une vis de blocage et une vis sans blocage ayant une tête, et une plaque (10, 110, 210) configurée pour la fixation d'un os ou d'os du milieu du pied, la plaque (10, 110, 210) ayant une surface supérieure (29) et une surface inférieure (27) avec une épaisseur généralement uniforme entre elles, **caractérisé en ce que** la plaque (10, 110, 210) a un profil qui comprend un premier côté latéral assemblé à un second côté latéral, dans lequel chacun parmi le premier côté latéral et le second côté latéral a un profil qui comprend une première languette d'extrémité (12, 14, 112, 114, 212, 214) qui définit une partie d'un cercle et une seconde languette d'extrémité (12, 14, 112, 114, 212, 214) qui définit une partie d'un cercle, dans lequel les première et seconde languettes d'extrémité (5, 12, 14, 112, 114, 212, 214) sont alignées le long d'un axe longitudinal de la plaque et assemblées par une section centrale, la première languette d'extrémité (12, 14, 112, 114, 212, 214) comprenant un trou de blocage (22, 122, 222) pour une vis de blocage, et la seconde languette d'extrémité (12, 14,, 112, 114, 212, 214) comprenant une fente de compression (20, 120, 220) ayant un axe long, la fente de compression (20, 120, 220) comprenant un bord latéral qui comprend un épaulement incliné qui s'incline vers le bas vers la surface inférieure de la plaque qui est mise en prise par la tête de la vis sans blocage afin de provoquer la translation de la vis sans blocage le long de l'axe long de la fente de compression (20, 120, 220) et vers le trou de blocage (22, 122, 222), le trou de blocage (22, 122, 222) et la fente de compression (20, 120, 220) sont alignés le long de l'axe longitudinal et une vis sans blocage interagissant avec la fente de compression pour provoquer la compression dans l'os associé vers le trou de blocage, à l'usage, et la plaque comprenant facultativement au moins un autre trou pour une broche de Kirschner, dans lequel la plaque comprend des courbures (23) entre la première languette d'extrémité et la seconde languette d'extrémité afin de former une section d'étranglement, dans lequel le trou de blocage (7, 22, 120, 220) comprend des filetages internes et la vis de blocage a une tête ayant des filetage externes de couplage.

2. Système de plaque selon la revendication 1, dans lequel la surface inférieure (27) peut faire face à un os et comprend un rayon de courbure le long de l'axe longitudinal.

3. Système de plaque selon la revendication 2, dans lequel la plaque (10) comprend une partie d'un cylindre.

4. Système de plaque selon la revendication 1, dans lequel la vis de blocage comprend un mécanisme de blocage variable.

5. Système de plaque dans lequel la plaque a un profil qui est un losange.

6. Système de plaque selon la revendication 1, dans lequel la plaque a un axe longitudinal et la surface inférieure peut faire face à un os et comprend un rayon de courbure le long de l'axe longitudinal.

7. Système de plaque selon la revendication 6, dans lequel la plaque a un axe transversal par rapport à l'axe longitudinal qui comprend également un rayon de courbure.

8. Système de plaque selon la revendication 1, dans lequel la fente de compression comprend un bord latéral qui s'incline de la surface supérieure vers la surface inférieure dans la direction du trou de blocage.

9. Système de plaque selon l'une quelconque des revendications précédentes, dans lequel la plaque a deux ensembles diagonaux de languettes (12, 14, 112, 114, 212, 214) opposées, dans lequel la longueur maximum de la plaque s'étend entre l'ensemble de languettes le plus long des deux ensembles diagonaux de languettes opposées.

10. Système de plaque selon l'une quelconque des revendications précédentes, dans lequel les trous de blocage (22, 122, 222) et les fentes de compression (20, 120, 220) du premier côté latéral et du second côté latéral sont décalés les uns par rapport aux autres.
